# EUROPÄISCHE PATENTSCHRIFT

(11) **EP 2 196 141 B1**
(45) Veröffentlichungstag und Bekanntmachung des Hinweises auf die Patenterteilung: **11.05.2011**
(21) Anmeldenummer: 09075540.6
(22) Anmeldetag: 03.12.2009
(51) Int. Cl.: A61B 5/022, A61B 8/04

(54) **Messgerät zur nicht-invasiven Langzeitmessung des Blutdruckes**
Measurement device for non-invasive long-term measurement of blood pressure
Appareil de mesure destiné à la mesure longue durée non invasive de la pression sanguine

(30) Priorität: 08.12.2008 DE 102008061018
(43) Veröffentlichungstag der Anmeldung: 16.06.2010
(73) Patentinhaber: Charité Universitätsmedizin Berlin, 10117 Berlin (DE)
(72) Erfinder: Affeld, Klaus, Prof. Dr., 10629 Berlin (DE)
(74) Vertreter: Neumann, Günter

(56) Entgegenhaltungen:
- EP-A1- 0 299 827
- WO-A1-2007/100107
- DE-U1- 29 807 158
- US-A- 4 127 114
- US-A1- 2005 085 728
- US-B1- 6 952 605

## Beschreibung

Die Erfindung betrifft ein Messgerät zur nicht-invasiven Langzeitmessung des Blutdruckes.

Die Aufgabe der nicht-invasiven Langzeitmessung des Blutdruckes ist es, den sich über den Tages- oder Tätigkeitsverlauf ständig ändernden Blutdruck in einem Zeitprofil zu messen. Die Messung soll dabei störungsfrei arbeiten, den Patienten nicht in seiner Tätigkeit behindern und ihn auch nicht belasten. Die Messung des Blutdruckes hat eine große gesundheitspolitische Bedeutung, da eine Veränderung des normalen Blutdrucks, wie Bluthochdruck, viele verhängnisvolle Folgen für den Patienten haben kann.

Stand der Technik ist heute immer noch die 1896 von Riva-Rocci erfundene Methode der Armmanschette, die wenig später von Korotkoff verbessert wurde. Seither sind über hundert Jahre vergangen, und trotz des großen Fortschritts der Medizintechnik auf fast allen anderen Gebieten sind auf dem Gebiet der Blutdruckmessung nur geringe Fortschritte erzielt worden.

Es ist gelungen, die Einzelmessung zu automatisieren, und man hat Geräte entwickelt, mit denen man über 24 Stunden ein Blutdruckprofil aus Einzelmessungen im Abstand von 15 Minuten gewinnt. Bei der Messung wird die Armmanschette mit Luft aufgepumpt, bis sie den Blutstrom in den Arm unterbindet, dann wird der Druck gezielt abgesenkt und das pulsweise Einströmen des Blutes in die wieder eröffneten Arterien wird durch Geräuschwirkung (Korotkoff-Methode), durch Volumenänderung oder durch die Messung der Blutgeschwindigkeit erfasst. Für den Patienten sind diese Verfahren belastend:
Beim Aufpumpen der Manschette werden die venösen Gefäße zuerst verschlossen, während durch die Arterien wegen des in ihnen herrschenden höheren Druckes noch Blut in das Gefäßbett strömt. Das verursacht ein unangenehmes Dehnungsgefühl. Hinzu kommen bei vielen Patienten ein als unangenehm empfundenes "Pochen" und ein Taubheitsgefühl im Arm.
Die Auswertung der Messung basiert auf der Annahme, dass die Armmanschette sich in Höhe des Herzens und der Arm in Ruhe befindet. Jede Tätigkeit des Patienten muss daher unterbrochen werden, da es sonst zu einer Fehlmessung kommt. Gleichfalls stört die Messung durch Geräuscheinwirkung und das erwähnte Druckgefühl die Nachtruhe der Patienten, wodurch die Ergebnisse verfälscht werden. Diese Nachteile haben dazu geführt, dass die 24-Stunden-Messung nicht die breite Anwendung gefunden hat, die auf Grund der Epidemiologie eigentlich erforderlich ist.

Das Problem der nicht-invasiven Langzeitmessung des Blutdrucks ist Gegenstand vieler Patentschriften geworden, in denen neue Lösungsansätze vorgeschlagen wurden.

In der Patentschrift "Wearable Blood Pressure Monitoring System" - Mühlsteff, 2008 WO 2008/004159 A3 wird eine Methode beschrieben, bei der das unangenehme Abklemmen venöser Gefäße vermieden werden kann. Das hier verwendete Messprinzip beruht auf der Abhängigkeit der Pulswellengeschwindigkeit vom Blutdruck. Die Pulswellengeschwindigkeit wird mit Hilfe der Bioimpedanz bestimmt, die durch hautnahe Elektroden gemessen wird. Jedoch besitzen alle arteriellen Gefäßwände einen Anteil glatter Muskulatur, die vom Zentralnervensystem gesteuert wird. Die Elastizität der Gefäße und daher auch die Pulswellengeschwindigkeit sind somit veränderlich. Damit können keine reproduzierbaren Ermittlungen des Blutdrucks erreicht werden und das Verfahren ist nicht geeignet, den Blutdruck zuverlässig zu messen.

In der Patentschrift "Device and method for the continuous non-invasive measurement of blood pressure" - Skrabal, 2006 US 0,195,034 A1 wird eine Methode beschrieben, die auf dem Prinzip der entspannten Arterienwand beruht. Die Messung des Blutdruckes wird bei dieser Methode an zwei Fingern vorgenommen. Beide Finger werden teilweise von einer Manschette umschlossen, die von einem schnellen Regler so gefüllt wird, dass der Manschettendruck dem Blutdruck folgt. Der Regler wird über einen Lichtplethysmographen gesteuert, der die gleichmäßige Füllung des Gefäßbettes regelt. Die Wand der Arterie ist also ohne Spannung, ein transmuraler Druck tritt nicht auf und das Gewebe wird mit einem Druck beaufschlagt, der gleich dem Blutdruck ist. Eine der Kammern dient zur Referenzdruckbestimmung. Dieses Messverfahren beruht auf einer Methode, die 1973 von Pe 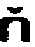 áz vorgestellt wurde. Es wurde später zu einem klinisch anwendbaren Verfahren entwickelt und ist für stationäre Messungen als Marke "Finapres" und für mobile Messungen als Marke "Portapres" bekannt geworden. Es ist für eine Langzeitmessung jedoch nicht geeignet, weil es im Widerspruch zur Physiologie steht: die Wand der Arterie ist für die Aufnahme der durch den Gefäßinnendruck verursachten Zugspannungen ausgebildet, damit das umliegende Gewebe drucklos ist. Dies ist hier umgekehrt: die Wand der Arterie ist ohne Spannung und es wirkt ein Druck auf das umgebende Gewebe. Dieser Druck ist größer als der Kapillardruck (etwa 30 mm Hg), und das Gewebe kann nicht mehr von Blut durchströmt werden. Bei langer Einwirkung des Druckes wird das Gewebe nekrotisch und stirbt ab. Bei vielen Messverfahren, die auf dem Prinzip der entspannten Arterienwand beruhen, vermeidet man deshalb lange Einwirkungszeiten, z. B. durch abwechselnde Messungen an mehreren Fingern. Dennoch führt das Messverfahren zu Fingerspasmen, ischämischen Schmerzen, Taubheitsgefühlen und zyanotischen Verfärbungen. Bei der von Skrabal vorgeschlagenen Methode ist eine Abwechslung der Messorte nicht vorgesehen. Neben den Einschränkungen der normalen Tagestätigkeit ist nicht ersichtlich, wie die Kammern in Bezug auf die Blutgefäße sicher am Körper befestigt werden sollen. Das Verfahren ist für die belastungsarme Langzeitmessung des Blutdruckes nicht geeignet.

In der Patentschrift "Non-invasive in vivo pressure measurement" - Madsen, 2003 US 6,547,734 wird eine Methode vorgestellt, bei der ein an der Körperoberfläche liegendes Gefäß mit einem Druck über eine flüssigkeitsgefüllte Kapsel beaufschlagt wird und gleichzeitig die Blutgeschwindigkeit im Gefäß mit einer Ultraschall-Dopplersonde erfasst und mit dem Drucksignal korreliert wird. Diese Messung wird von einem Arzt durchgeführt, der die kombinierte Druck-Geschwindigkeits-Sonde mit der Hand über dem Blutgefäß in Position bringt und dort hält. Diese Methode ist damit für eine Langzeitmessung des Blutdruckes nicht geeignet, da sie keine Lehre enthält, wie eine konstante Position des Apparates zum Gefäß gewährleistet werden kann. Da jede Verschiebung der Sonde sowohl eine Veränderung des Drucks als auch eine Veränderung der optimalen Position der Geschwindigkeitsmesssonde zum Gefäß mit sich bringt, kann eine Messung über eine längere Zeit nicht durchgeführt werden.

In der Patentschrift "Blood pressure monitoring technique which utilizes a patient's supraorbital artery" - Bobo, 1993 US 00,523,034 A wird eine Methode beschrieben, den Druck an der Arteria supraorbitalis, einem kleinen Gefäß an der Stirn über den Augenbrauen, zu messen. Es soll ein starres Gehäuse mit einem Ballon so über dem Blutgefäß platziert werden, dass der Puls auf die Luft oder ein Fluid im Ballon übertragen werden kann. Nach den bekannten Methoden der oszillometrischen Blutdruckmessung wird dann der Puls ausgewertet. Die Befestigung des starren Gehäuses des Ballons auf der Haut erfolgt durch ein Klebepflaster. Da das ausgewählte Blutgefäß sehr klein ist, ist auch der Puls sehr schwach. Eine kleine Veränderung der Position des starren Gehäuses führt zu Störungen, weshalb die Befestigung durch ein elastisches Pflaster die nötige Unverschiebbarkeit nicht gewährleisten kann. In der nachfolgenden Patentschrift vom gleichen Erfinder "Blood pressure monitoring pad assembly and method" - Bobo, 1996 US 5,586,555 wird ein verbessertes starres Gehäuse beschrieben, dessen Hauptaufgabe die Hauteinleitung der durch das Aufpumpen des Ballons entstehenden Kräfte ist. Auch hier wird ein elastisches Klebepflaster verwendet, weil es biegsam sein muss, wenn es zur Entfernung nach der Messung an einem Ende beginnend durch eine Schälspannung abgezogen werden soll. Mit einem elastischen Klebepflaster ist eine unverschiebbare und sichere Platzierung des Ballons über dem Blutgefäß nicht möglich.

In der Patentschrift "Noninvasive temporal artery blood pressure sensor assembly" - Borsanyi 1992 PCT/US92/07279 wird ein Apparat beschrieben, der mit Hilfe eines Stirnbandes über der Arteria temporalis superficialis angebracht und den Blutdruck mit Hilfe einer oszillometrischen Messmethode erfassen soll. Die Arterie soll mit Hilfe eines Ballons okkludiert werden, der gleichzeitig die Druckveränderungen erfassen kann.
Mit einem Stirnband kann ein Sensor am Kopf nur unverschiebbar positioniert werden, wenn das Stirnband sehr fest angezogen wird. Dann tritt aber eine Ischämie der Kopfhaut ein und eine Dauermessung ist mit dieser Anordnung nicht möglich.

In der Patentschrift EP 0299827 A wird ein Messgerät zur nicht-invasiven Messung des Blutdruckes mit einer Vorrichtung zur Okklusion des Blutgefäßes und einem Ultraschalldopplersensor beschrieben.

Allen Methoden ist gemein, dass sie entweder auf einem nicht wirksamen Messprinzip beruhen oder es nicht gestatten, die Messsonde zuverlässig und für den Patienten schonend sicher in der Nähe des Blutgefäßes zu fixieren.

Der Erfindung liegt daher die Aufgabe zugrunde, die belastungsarme nicht-invasive Langzeitmessung des Blutdruckes an einem dicht unter der Körperoberfläche liegenden Blutgefäß mit einer Vorrichtung zur Okklusion des Blutgefäßes und einem Ultraschalldopplersensor sowie einem Verfahren auf eine neue und wirksame Weise zu ermöglichen. Erfindungsgemäß wird die Aufgabe durch ein Messgerät zur nicht-invasiven Blutdruckmessung an einem dicht unter der Körperoberfläche liegenden Blutgefäß mit einer Vorrichtung zur Okklusion des Blutgefäßes und einem Ultraschalldopplersensor dadurch gelöst, dass die Vorrichtung zur Okklusion des Blutgefäßes ein in seiner Längsachse verschiebbarer und steuerbarer Stempel mit einem im Dopplermodus betriebenen Ultraschallkristall zur Ermittlung der Blutgeschwindigkeit im Blutgefäß ist, ein auf der Körperoberfläche über dem Blutgefäß anhaftbares Klebeelement den Stempel aufnimmt, der mit einem Stellmotor zum vertikalen Verschieben und einem Kraftmesssensor verbunden ist und das Messgerät eine Zuleitung zur Zuführung eines Mittels zum Lösen des Klebeelements von der Körperoberfläche besitzt.

Eine bevorzugte Ausführungsform des erfindungsgemäßen Messgerätes sieht vor, dass das Klebeelement aus einem starren mikroporösen Werkstoff besteht.

Gemäß einer weiteren Ausführungsform der Erfindung ist das Klebeelement, außer an seiner Körperkontaktfläche, an allen übrigen Flächen mit einer lösungsmitteldichten und lösungsmittelbeständigen Schicht überzogen.

Nach einer besonders bevorzugten Ausführungsform des Messgerätes weist das Klebeelement unter seiner Körperkontaktfläche eine Folie auf, die vorzugsweise mit einem Antihaftmaterial beschichtet ist und die nach Positionierung des Klebeelements auf der Körperoberfläche über dem Blutgefäß abziehbar ist.

Vorzugsweise ist der Stellmotor mittels eines Tragelementes am Klebeelement angeordnet.

Eine weitere Ausführungsform der Erfindung sieht vor, dass der Stempel ein der Körperoberfläche zugewandtes Ende aus einem leicht verformbaren und den Schall gut leitenden Material aufweist.

Bevorzugt weist das Messgerät eine Zuleitung zum Aufbringen eines Ultraschallkontaktgels aus einem Reservoir auf die Körperoberfläche auf, wobei die Zuleitung vorzugsweise im oder am Stempel angeordnet ist.

Erfindungsgemäß wird die Aufgabe des Weiteren durch ein Verfahren gelöst, bei dem auf der Körperoberfläche ein Modul mit einem stempelförmigen Element mittels einer aus einem Antihaftmittel bestehenden oder mit einem Antihaftmittel beschichteten und unter dem Modul abziehbar angeordneten Folie positioniert und nach dem Abziehen der Folie durch Klebung auf der Haut fixiert wird, anschließend das stempelförmige Element mittels einer Steuerung in Richtung des Blutgefäßes bis zu dessen Okklusion vorwärts bewegt wird, danach das stempelförmige Element langsam zurückbewegt wird, bis mittels eines Ultraschallkristalles erste Blutgeschwindigkeitsspitzen gemessen werden und so durch die gleichzeitige Messung der auf das Blutgefäß wirkenden Kraft des stempelförmigen Elementes eine Messung des systolischen Blutdruckes erfolgt und schließlich das stempelförmige Element weiter langsam zurückbewegt, bis mit dem Ultraschallkristall ein zeitlicher Verlauf der Blutgeschwindigkeit erfasst wird, bei dem die Blutgeschwindigkeit kurzzeitig den Wert Null erreicht, wodurch bei gleichzeitiger Korrelation mit der auf das Blutgefäß wirkenden Kraft des stempelförmigen Elementes der diastolische Blutdruck gemessen wird.

Gemäß einer bevorzugten Ausführungsform des Verfahrens wird mittels Lagesensoren die Differenz zwischen der Höhe des Klebeelements und der Höhe des Herzens ermittelt und mit der daraus gewonnenen hydrostatischen Druckdifferenz die Messung des Blutdruckes korrigiert.

Mit dem erfindungsgemäßen Messgerät und dem Verfahren kann auf vorteilhafte Art und Weise, zuverlässig und für den Patienten belastungsarm die nicht-invasive Langzeitmessung des Blutdruckes an einem dicht unter der Körperoberfläche liegenden Blutgefäß durchgeführt werden.

Im Folgenden sollen die Erfindung und ihre Vorzüge an Hand von Zeichnungen näher erläutert werden. Es zeigen:
- **Figur 1**: eine schematische Darstellung des Messprinzips mit einem in seiner Längs- achse verschiebbaren und steuerbaren Stempel
- **Figur 2**: das Abziehen der Schutzfolie bei der Aufklebung des Klebeelements
- **Figur 3**: den Schnitt durch das Klebeelement
- **Figur 4**: den zeitlichen Verlauf der Blutgeschwindigkeit bei verschiedenen Zuständen der Okklusion.

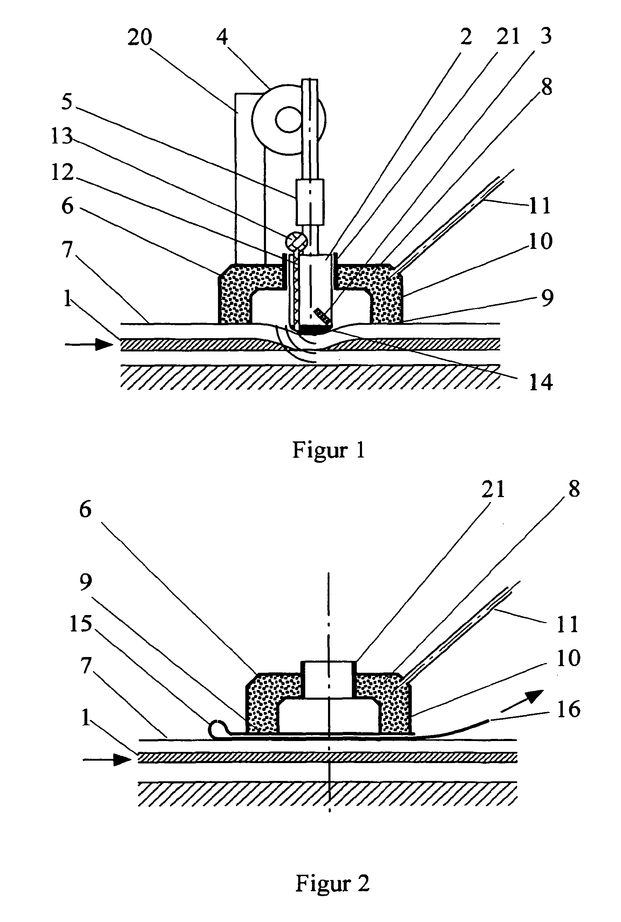

**Fig. 1** zeigt die erfindungsgemäße Anordnung der einzelnen Elemente. Das dicht unter der Körperoberfläche liegende Blutgefäß 1 wird zu Beginn der Messung mit einem Ultraschallkristall 3 lokalisiert, damit das Klebeelement 6 in die richtige Position gebracht werden kann. Das Klebeelement 6 ist an seiner Unterseite, der Hautkontaktfläche 9, mit einem Kleber beschichtet und wird durch Andrücken auf der Haut befestigt. Der in seiner Längsachse verschiebbare und steuerbare Stempel 2 enthält den Ultraschallkristall 3, der im Dopplermodus betrieben wird und in bekannter Weise die Blutgeschwindigkeit misst. Der in seiner Längsachse verschiebbare und steuerbare Stempel 2 wird durch einen fest mit dem Klebeelement 6 verbundenen Stellmotor 4 gegen die Körperoberfläche bewegt und okkludiert auf diese Weise erst teilweise, dann vollständig das Blutgefäß 1. Der Stempel 2 wird in einer Führung 21 geführt. An seiner unteren, der Haut zugewandten Seite besteht er aus einem flexiblen Material, das sich der Haut anpassen kann und das den Schall gut leitet. Solches Material ist beispielsweise ein Silikonkunststoff mit einem hohen Weichmacheranteil mit einer gelartigen Struktur, wie es von Implantaten her bekannt ist. Durch die Bewegung des Stempels 2 in die Richtung des Blutgefäßes 1 wird dieses verengt, und es verändert sich die Blutgeschwindigkeit in charakteristischer Weise. Man kann durch die gleichzeitige Messung der Stempelkraft mit dem Kraftsensor 5 und des Blutflusses mit dem Ultraschallkristall 3 sowohl den systolischen wie auch den diastolischen Blutdruck ermitteln. Zur Verbesserung der Geschwindigkeitsmessung kann durch die Zuleitung 12 ein Ultraschallgel aus einem Reservoir (13) auf die Haut aufgebracht werden. Nach der Messung bewegt sich der in seiner Längsachse verschiebbare und steuerbare Stempel 2 wieder zurück und übt dann keinen Druck auf das Körpergewebe aus.

Auf diese Weise kann das Körpergewebe normal durchblutet werden und es treten keine ischämischen Beschwerden auf. Die Messung kann dann in bestimmten Zeitabständen wiederholt werden und es wird dadurch möglich, ein Zeitprofil des Blutdruckes zu ermitteln. Aus dem Geschwindigkeitssignal kann außerdem die Pulsfrequenz ermittelt werden. Nach der Messung eines Tagesprofils wird das Klebeelement 6 wieder entfernt. Es ist aus einem mikroporösen Werkstoff gefertigt und an allen Außenflächen außer der Hautkontaktfläche 9 mit einem lösungsmitteldichten Werkstoff (10) überzogen. Zudem besitzt er eine Zuführung 11, durch die ein Lösungsmittel für den Kleber eingeführt werden kann. Nach kurzer Einwirkungszeit kann das vorher fest mit der Haut verklebte Klebeelement kraftlos entfernt werden.

**Fig. 2** zeigt das Klebeelement 6 vor der Klebung. Als Kleber eignet sich ein lösungsmittelfreier Kontaktkleber, wie er für Klebefolien und für Heftpflaster verwendet wird. Ein weiterer Kleber kann ein Methacrylatkleber sein, der auch als Sekundenkleber bekannt ist. Er wird kurz vor dem Gebrauch auf die Klebefläche aufgebracht und mit der Abziehfolie 15 abgedeckt. Die Abziehfolie 15 bedeckt die Klebefläche 9 und ermöglicht ein leichtes Verschieben des Klebeelements 6 auf der Haut 7, bis die optimale Position mit der Hilfe des Ultraschallkristalls 3 gefunden ist. Die Abziehfolie 15 besteht aus einem dünnen Kunststoff mit Antihafteigenschaften, wie sie beispielsweise durch eine Beschichtung mit den Kunststoffen PTFE oder Silikonkautschuk erzielt werden können. Diese Antihafteigenschaften bewirken, dass die Abziehfolie 15 in der dargestellten Weise an ihrem Ende 16 unter dem Klebeelement 6 hervorgezogen und von ihm abgeschält werden kann. Dabei wird die Hautkontaktfläche 9 frei und somit wirksam. Somit wird die Klebung in der vorher gefundenen optimalen Position wirksam.

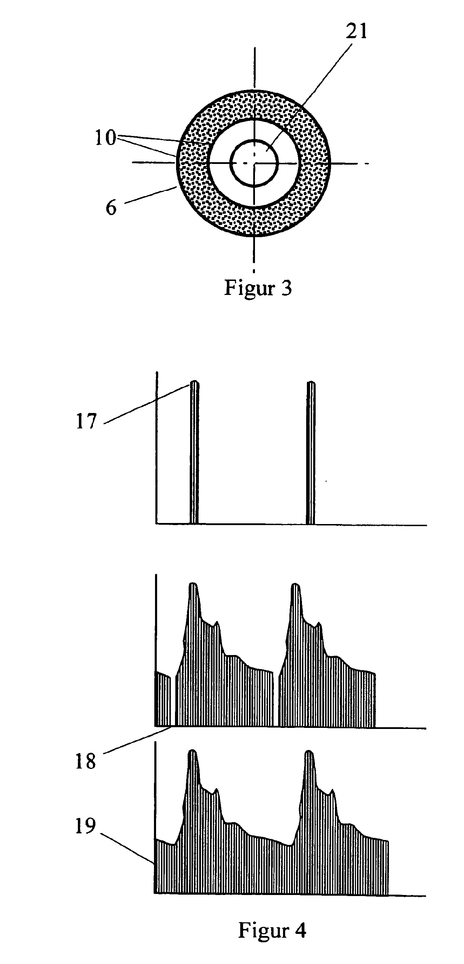

**Fig. 3** zeigt das Klebeelement 6 in einem Schnitt. Der mikroporöse starre Werkstoff kann beispielsweise ein poröses Metall sein, wie es als Metapor bekannt geworden ist. Es besitzt eine ausreichende Festigkeit, um die Führung für den Stempel 2 ohne Verformung zu halten. Durch die Poren kann das Lösungsmittel nach der Messung zur Hautkontaktfläche 9 vordringen und dort die Klebung mit der Haut beschwerdefrei lösen.

**Fig. 4** zeigt den zeitlichen Verlauf der Blutgeschwindigkeit bei unterschiedlichen Zuständen der Okklusion. Nach vollkommener Okklusion verschwindet das Blutgeschwindigkeitssignal. Bei einem Nachlassen der Okklusion erscheinen kurze Spitzenwerte 17 der Blutgeschwindigkeit, dies wird mit der Stempelkraft korreliert und ergibt den systolischen Blutdruck. Bei weiterem Nachlassen der Stempelkraft erscheint mehr und mehr vom Blutgeschwindigkeitssignal 18, das nur noch in kurzen Zeiten den Wert Null erreicht. Dies wird mit der Stempelkraft korreliert und ergibt den diastolischen Blutdruck. Bei weiterem Nachlassen der in seiner Längsachse verschiebbaren und steuerbaren Stempelkraft wird der Blutfluss nicht mehr behindert und es erscheint der ungestörte zeitliche Verlauf der Blutgeschwindigkeit 19.

## Patentansprüche

1. Messgerät zur nicht-invasiven Langzeitmessung des Blutdruckes an einem dicht unter der Körperoberfläche (7) liegenden Blutgefäß (1) mit einer Vorrichtung zur Okklusion des Blutgefäßes (1) und einem Ultraschalldopplersensor,
wobei die Vorrichtung zur Okklusion des Blutgefäßes (1) ein in seiner Längsachse verschiebbarer und steuerbarer Stempel (2) mit einem im Dopplermodus betriebenen Ultraschallkristall (3) zur Ermittlung der Blutgeschwindigkeit im Blutgefäß (1) ist, **dadurch gekennzeichnet, dass**
- ein auf der Körperoberfläche (7) über dem Blutgefäß (1) anhaftbares Klebeelement (6) den Stempel (2) aufnimmt;
- der Stempel (2) mit einem Stellmotor (4) zum vertikalen Verschieben und einem Kraftmesssensor (5) verbunden ist und
- das Messgerät eine Zuleitung (11) zur Zuführung eines Mittels zum Lösen des Klebeelements (6) von der Körperoberfläche (7) besitzt.

2. Messgerät nach Anspruch 1, **dadurch gekennzeichnet, dass** das Klebeelement (6) aus einem starren mikroporösen Werkstoff (8) besteht.

3. Messgerät nach Anspruch 1 oder 2, **dadurch gekennzeichnet, dass** das Klebeelement (6) außer an seiner Körperkontaktfläche (9) an allen übrigen Flächen mit einer lösungsmitteldichten und lösungsmittelbeständigen Schicht (10) überzogen ist.

4. Messgerät nach einem der Ansprüche 1 bis 3, **dadurch gekennzeichnet, dass** das Klebeelement (6) unter seiner Körperkontaktfläche (9) eine Folie (15) aufweist, die vorzugsweise mit einem Antihaftmaterial beschichtet ist, die nach Positionierung des Klebeelements (6) auf der Körperoberfläche (7) über dem Blutgefäß (1) abziehbar ist.

5. Messgerät nach einem der Ansprüche 1 bis 4, **dadurch gekennzeichnet, dass** der Stellmotor (4) mittels eines Tragelementes (20) am Klebeelement (6) angeordnet ist.

6. Messgerät nach einem der Ansprüche 1 bis 5, **dadurch gekennzeichnet, dass** der Stempel (2) ein der Körperoberfläche (7) zugewandtes Ende (14) aus einem leicht verformbaren und den Schall gut leitenden Material aufweist.

7. Messgerät nach einem der Ansprüche 1 bis 6, **dadurch gekennzeichnet, dass** es eine Zuleitung (12) zum Aufbringen eines Ultraschallkontaktgels aus einem Reservoir (13) auf die Körperoberfläche (7) aufweist, wobei die Zuleitung (12) vorzugsweise im oder am Stempel (2) angeordnet ist.

8. Verfahren zur nicht-invasiven Langzeitmessung des Blutdruckes an einem dicht unter der Körperoberfläche liegenden Blutgefäß einer Person, **dadurch gekennzeichnet, dass**
- auf der Körperoberfläche ein Modul mit einem stempelförmigen Element mittels einer aus einem Antihaftmittel bestehenden oder mit einem Antihaftmittel beschichteten und unter dem Modul abziehbar angeordneten Folie positioniert und nach dem Abziehen der Folie durch Klebung auf der Haut fixiert wird,
- das stempelförmige Element mittels einer Steuerung in Richtung des Blutgefäßes bis zu dessen Okklusion vorwärts bewegt wird,
- danach das stempelförmige Element langsam zurückbewegt wird, bis mittels eines Ultraschallkristalles erste Blutgeschwindigkeitsspitzen gemessen werden und so durch die gleichzeitige Messung der auf das Blutgefäß wirkenden Kraft des stempelförmigen Elementes eine Messung des systolischen Blutdruckes erfolgt,
- anschließend das stempelförmige Element weiter langsam zurückbewegt, bis mit dem Ultraschallkristall ein zeitlicher Verlauf der Blutgeschwindigkeit erfasst wird, bei dem die Blutgeschwindigkeit kurzzeitig den Wert Null erreicht, wodurch bei gleichzeitiger Korrelation mit der auf das Blutgefäß wirkenden Kraft des stempelförmigen Elementes der diastolische Blutdruck gemessen wird.

9. Verfahren nach Anspruch 8, **dadurch gekennzeichnet, dass** mittels Lagesensoren die Differenz zwischen der Höhe des Klebeelements und der Höhe des Herzens ermittelt wird und mit der daraus gewonnenen hydrostatischen Druckdifferenz die Messung des Blutdruckes korrigiert wird.

## Claims

1. Measurement device for the non-invasive long-term measurement of the blood pressure of a blood vessel (1) located close beneath the body surface (7) having an apparatus for occlusion of the blood vessel (1) and an ultra-sonic Doppler-sensor, whereas
the apparatus for occlusion of the blood-vessel (1) is an indenter (2) movable and controllable along its longitudinal axis with an ultra-sonic crystal (3) operated in Doppler-mode for detection of the blood velocity in the blood vessel (1),
**characterized in that**
- an adhesive element (6) on the body surface (7) above the blood vessel (1) holds the indenter (2);
- the indenter (2) is connected to an actuator (4) for vertically displacement and a force measurement sensor (5) and
- the measurement device comprises a supply line (11) for supplying a means to release the adhesive element (6) from the body surface (7).

2. Measurement device according to Claim 1,
**characterized in that**
the adhesive element (6) consists of a micro-porous material (8).

3. Measurement device according to Claim 1 or 2,
**characterized in that**
the adhesive element (6) except for the body contact surface (9) is covered on all remaining surfaces with a solvent-tight and solvent-resistant coating (10).

4. Measurement device according to any one of Claims 1 to 3,
**characterized in that**
the adhesive element (6) comprises below its body contact surface (9) a foil, which is preferably coated with an anti-adhesive material, which is peelable after positioning of the adhesive element (6) on the body surface (7) above the blood vessel (1).

5. Measurement device according to any one of Claims 1 to 4,
**characterized in that**
the actuator (4) is arranged via a carrier element (2) on the adhesive element (6).

6. Measurement device according to any one of Claims 1 to 5,
**characterized in that**
the indenter (2) comprises a body surface (7) facing end (14) consisting of an easy deformable and sound well conducting material.

7. Measurement device according to any one of Claims 1 to 6,
**characterized in that**
it comprises a supply line (12) for application of an ultra-sonic contact gel from a reservoir (13) to the body surface (7), whereas the supply line (12) preferably is arranged in or on the indenter (2).

8. Method for non-invasive long-term measurement of the blood-pressure of a blood vessel close beneath the body surface of a person,
**characterized in that**,
- on the body surface a module with an indenter-shaped element is positioned via a foil consisting of anti-adhesive material or which is coated with an anti-adhesive material, which is detachably located below the module and after peeling off the foil is fixed on the skin by adhesion,
- the indenter shaped element is moved forwardly by means of a controller into the direction of the blood vessel until its occlusion,
- after that the indenter shaped element is retracted slowly, until first blood velocity peaks can be measured via an ultra-sonic crystal and so by the concurrent measurement of the force of the indenter shaped element acting onto the blood vessel a measurement of systolic blood pressure takes place,
- then the indenter shaped element is further retracted slowly until a chronologic sequence of the blood velocity is detected, in which the blood velocity momentary reaches the value zero, by what under concurrent correlation with the force of the indenter shaped element acting on the blood vessel the diastolic blood pressure is measured.

9. Method according to Claim 8,
**characterized in that**
via position sensors the difference between height of the adhesive element and the height of the heart is determined and the measurement of blood pressure is corrected with the hydrostatic pressure difference derived therefrom.

## Revendications

1. Appareil de mesure destiné à la mesure longue durée non invasive de la pression sanguine au niveau d'un vaisseau sanguin (1) se trouvant directement sous la surface du corps (7) à l'aide d'un dispositif d'occlusion du vaisseau sanguin (1) et d'un capteur Doppler à ultrasons, le dispositif d'occlusion du vaisseau sanguin (1) étant un tampon applicateur (2) pouvant être commandé et déplacé sur son axe longitudinal qui est équipé de cristaux ultrasoniques (3) commandés en mode Doppler pour la détermination de la vitesse sanguine dans le vaisseau sanguin (1), **caractérisé en ce que**
- le tampon applicateur (2) est logé dans un élément adhésif (6) pouvant coller sur la surface du corps (7) au-dessus du vaisseau sanguin (1),
- le tampon applicateur (2) est relié à un servomoteur (4) pour son déplacement vertical et à un capteur de mesure de force (5) et
- l'appareil de mesure possède une conduite d'amenée (11) pour l'amenée d'un agent destiné à détacher l'élément adhésif (6) de la surface du corps (7).

2. Appareil de mesure selon la revendication 1, **caractérisé en ce que** l'élément adhésif (6) se compose d'un matériau (8) rigide microporeux.

3. Appareil de mesure selon la revendication 1 ou 2, **caractérisé en ce que** l'élément adhésif (6) est recouvert sur toutes ses faces, sauf sur sa surface en contact avec le corps (9), d'une couche (10) étanche aux solvants et résistante aux solvants.

4. Appareil de mesure selon l'une quelconque des revendications 1 à 3, **caractérisé en ce que** l'élément adhésif (6) présente, sous sa surface en contact avec le corps (9), un film (15) enduit de préférence d'un matériau antiadhésif qui peut être enlevé après le positionnement de l'élément adhésif (6) sur la surface du corps (7) au-dessus du vaisseau sanguin (1).

5. Appareil de mesure selon l'une quelconque des revendications 1 à 4, **caractérisé en ce que** le servomoteur (4) est disposé sur l'élément adhésif (6) au moyen d'un élément support (20).

6. Appareil de mesure selon l'une quelconque des revendications 1 à 5, **caractérisé en ce que** le tampon applicateur (2) présente une extrémité (14) tournée vers la surface du corps (7) constituée d'un matériau légèrement déformable et bon conducteur de sons.

7. Appareil de mesure selon l'une quelconque des revendications 1 à 6, **caractérisé en ce qu'**il présente une conduite d'amenée (12) pour l'application sur la surface du corps (7) d'un gel de contact ultrasonique venant d'un réservoir (13), ladite conduite d'amenée (12) étant disposée de préférence dans ou sur le tampon applicateur (2).

8. Procédé pour la mesure longue durée non invasive de la pression sanguine au niveau d'un vaisseau sanguin d'une personne se trouvant directement sous la surface du corps, **caractérisé en ce que**
- un module muni d'un élément en forme de tampon applicateur est positionné sur la surface du corps au moyen d'un film constitué d'un antiadhésif ou recouvert d'un agent antiadhésif ou disposé sous le module de manière à pouvoir être retiré et est fixé sur la peau par collage après avoir retiré le film,
- l'élément en forme de tampon applicateur est déplacé vers l'avant au moyen d'une commande en direction du vaisseau jusqu'à son occlusion,
- ensuite, l'élément en forme de tampon applicateur revient lentement en position jusqu'à la mesure des premières pointes de vitesse sanguine à l'aide des cristaux ultrasoniques et jusqu'à la mesure de la pression sanguine systolique grâce à la mesure simultanée de la force de l'élément en forme de tampon applicateur agissant sur le vaisseau sanguin,
- pour finir, l'élément en forme de tampon applicateur continue de revenir lentement en position jusqu'à la détermination, à l'aide des cristaux ultrasoniques, d'une courbe de temps de la vitesse sanguine pour laquelle la vitesse sanguine atteint la valeur zéro pour une courte durée, ce qui permet de mesurer la pression sanguine diastolique par corrélation simultanée avec la force de l'élément en forme de tampon applicateur agissant sur le vaisseau sanguin.

9. Procédé selon la revendication 8, **caractérisé en ce que** des capteurs de position permettent de déterminer la différence entre la hauteur de l'élément adhésif et la hauteur du coeur et la différence de pression hydrostatique en résultant permet de corriger la mesure de la pression sanguine.
